# EUROPEAN PATENT SPECIFICATION

(11) **EP 3 823 599 B1**
(45) Date of publication and mention of the grant of the patent: **28.08.2024**
(21) Application number: 19759061.5
(22) Date of filing: 18.07.2019
(51) Int. Cl.: A61K 9/20, A61K 31/519

(54) **PHARMACEUTICAL COMPOSITIONS WITH A CDC7 INHIBITOR**
PHARMAZEUTISCHE ZUSAMMENSETZUNGEN MIT EINEM CDC7-INHIBITOR
COMPOSITIONS PHARMACEUTIQUES COMPRENANT UN INHIBITEUR DE CDC7

(30) Priority: 19.07.2018 US 201862700384 P
(43) Date of publication of application: 26.05.2021
(73) Proprietor: Takeda Pharmaceutical Company Limited, 1-1, Doshomachi 4-chome, Chuo-ku Osaka-Shi, Osaka 541-0045 (JP)
(72) Inventor: GAO, Yijie, Cambridge, Massachusetts 02139 (US)
(74) Representative: Harris, Jennifer Lucy
(86) International application number: PCT/JP2019/029113
(87) International publication number: WO 2020/017666

(56) References cited:
- WO-A1-2017/172565
- US-B2- 8 722 660

## Description

### TECHNICAL FIELD

The present disclosure relates to pharmaceutical compositions comprising Compound 1 and/or tautomers thereof, or a pharmaceutically acceptable salt or hydrate thereof.

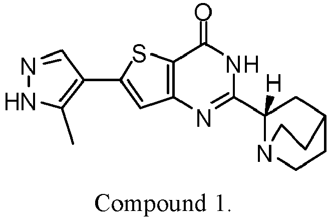

### BACKGROUND

Cdc7 is an evolutionally well-conserved serine/threonine kinase and plays an important role in the initiation of DNA replication (EMBO J. 1999, 18(20), p.5703-5713). The kinase activity of Cdc7 is controlled by binding with its activating partner thereof. From the late stage of G1 phase to S phase, Cdc7 forms a complex with Dbf4 (also known as ASK) and phosphorylates Cdc7 substrate to control transition from the G1 phase to the S phase (J Cell Physiol. 2002, 190(3), p.287-296). Furthermore, recent studies have reported that Cdc7 plays important roles in both DNA replication and DNA damage signaling pathways (Oncogene. 2008, 27(24), p.3475-3482).

Cdc7 kinase has received attention as an attractive target in cancer treatments. Overexpression of Cdc7 is observed in clinical tumors such as breast cancer, colorectal cancer, and lung cancer, and many cancer cell lines (Neoplasia. 2008, 10(9), p.920-931). In some cancer cell lines, an increase in chromosomal copy number of an activating factor, Dbf4, is found. Interestingly, a cancer cell line and an untransformed fibroblast cell line show different responses to suppression of Cdc7 expression using siRNA. The suppression of Cdc7 expression using siRNA causes the S phase arrest in cancer cell lines and induces apoptosis, whereas in normal cells it induces the G1 phase arrest in a p53 activity-dependent manner (Cancer Res. 2004, 64(19), p.7110-7116). Furthermore, Cdc7 kinase is activated in the cells under replication stress, and apoptosis induced by hydroxyurea and etoposide increases in the Cdc7 down-regulated cells (J Biol Chem. 2007, 282(1), p.208-215). Thus, a Cdc7 inhibitor, as a single agent or in combination with other chemotherapeutic agents, is useful for a selective cancer treatment.

### SUMMARY

In one aspect, the present disclosure provides a tablet comprising:
from about 10 wt% to about 30 wt% Compound 1 and/or tautomers thereof or a pharmaceutically acceptable salt or hydrate thereof 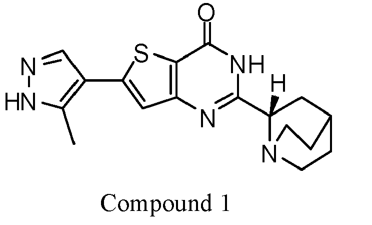
from about 3 wt% to about 10 wt% of silicified microcrystalline cellulose (SMCC),
from about 5 wt% to about 20 wt% of mannitol,
from about 0.25 wt% to about 2 wt% of polyvinylpyrrolidone (PVP),
from about 25 wt% to about 80 wt% of anhydrous lactose,
from about 2 wt% to about 3 wt% of croscarmellose sodium, and
from about 1 wt% to about 2 wt% of magnesium stearate.

In some embodiments, the tablet is produced by a method comprising dry granulation process.

In some aspects, a tablet of the present disclosure comprises an intra-granular component and an extra-granular component,
wherein the intra-granular component comprises:
   from about 40 wt% to about 60 wt% of Compound 1 and/or tautomers thereof or a pharmaceutically acceptable salt or hydrate thereof
   from about 10 wt% to about 20 wt% of SMCC,
   from about 20 wt% to about 40 wt% of mannitol,
   from about 1 wt% to about 3 wt% of PVP,
   from about 1 wt% to about 3 wt% of croscarmellose sodium, and
   from about 0.5 wt% to about 2 wt% of magnesium stearate; and
wherein the extra-granular component comprises:
   from about 90 wt% to about 98 wt% of anhydrous lactose,
   from about 2 wt% to about 5 wt% of croscarmellose sodium, and
   from about 1 wt% to about 3 wt% of magnesium stearate.

In some embodiments, the weight ratio of the intra-granular component to the extra granular component is from about 1:3 to about 2:1.

In some embodiments, the tablet is produced by a method comprising dry granulation process.

In some embodiments, a tablet of the present disclosure comprises:

| | |
|---|---|
| Compound 1 and/or tautomers thereof or a pharmaceutically acceptable salt or hydrate thereof | 12.5 wt% |
| Mannitol | 7.5 wt% |
| SMCC | 3.75 wt% |
| PVP | 0.5 wt% |
| Croscarmellose sodium | 2.5 wt% |
| MgSt | 1.25 wt% |
| Anhydrous lactose | 72 wt% |

In some embodiments, a tablet of the present disclosure comprises:

| | |
|---|---|
| Compound 1 and/or tautomers thereof or a pharmaceutically acceptable salt or hydrate thereof | 25 wt% |
| Mannitol | 15 wt% |
| SMCC | 7.5 wt% |
| PVP | 1 wt% |
| Croscarmellose sodium | 3 wt% |
| MgSt | 1.5 wt% |
| Anhydrous lactose | 47 wt% |

### BRIEF DESCRIPTION OF FIGURES

Fig. 1 is a plot of the compressibility of the drug substance and filler candidates.
Figs. 2A-C are plots of tablet tensile strength, porosity, and flow factor of preblend and dry granule. In Fig. 2C, when the compression pressure is 0 MPa, the point means Flow Factor of preblend. And, when the compression pressure is greater than 0 MPa, the point means Flow Factor of dry granule made under condition of the compression pressure.

### DETAILED DESCRIPTION

The technical information set out below may in some respects go beyond the scope of the present invention, which is defined by the appended claims. The additional technical information is provided to place the present invention in a broader technical context and to illustrate possible related technical developments

The present disclosure relates to pharmaceutical compositions comprising Compound 1 and/or tautomers thereof, or a pharmaceutically acceptable salt or hydrate thereof.

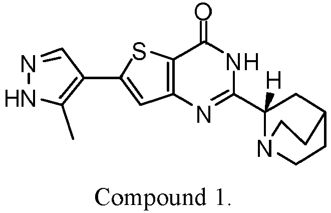

### Definitions

Unless defined otherwise, all technical and scientific terms used herein have the same meaning as commonly understood by one of ordinary skill in the art to which this disclosure belongs. Accordingly, the following terms are intended to have the following meanings.

As used in the specification and claims, the singular form "a", "an" and "the" includes plural references unless the context clearly dictates otherwise.

As used herein "compressible filler" refers to a pharmaceutical filler that has a high tensile strength when compressed into a tablet. In some embodiments, a compressible filler has a tensile strength greater than or equal to 3 MPa when compressed into a tablet at a compression pressure of 200 MPa. Non-limiting examples of compressible fillers are silicified microcrystalline cellulose (SMCC) and anhydrous lactose. Other forms of microcrystalline cellulose (MCC) and lactose, as well as certain forms of starch, with high compressibility are also suitable non-limiting examples of compressible fillers.

For example, when compressed into a tablet at a compression pressure of 200 MPa, SMCC has a tensile strength of about 9.5 MPa and anhydrous lactose has a tensile strength of about 5 MPa. Other filler materials having good compressibility, resulting in high tensile strength upon tablet compression, are known to a person of skill in the art.

As used herein "low-compressibility filler" refers to a pharmaceutical filler that has a low tensile strength when compressed into a tablet. In some embodiments, a low-compressibility filler has a tensile strength less than 3 MPa when compressed into a tablet at a compression pressure of 200 MPa. A non-limiting example of a low-compressibility filler is mannitol. For example, when compressed into a tablet at a compression pressure of 200 MPa, mannitol has a tensile strength of about 2 MPa. Other filler materials having low compressibility, resulting in low tensile strength upon tablet compression, are known to a person of skill in the art. For example, low compressibility forms of lactose may be used.

As used herein an "intra-granular" excipient for tablet refers to an excipient that is present in a component of the tablet formed by compression of granules. For example, "intra-granular compressible filler" ("IGCF") in a tablet refers to compressible filler that was formed into granules (e.g., together with other intra-granular excipients), which granules were compressed into the tablet.

As used herein an "extra-granular" excipient refers to an excipient that is present in a component of the tablet formed by compression of non-granulated material. For example, "extra-granular filler" refers to filler that was not granulated, which was compressed into the tablet. For example, the extra-granular excipients are added to the granules (containing intra-granular excipients), which together are compressed into a tablet having intra-granular and extra-granular regions.

As used herein "dry granulation" refers to a process of forming granules, i.e., dry granules, without using a liquid to aid in granulation. In a typical dry granulation process, a dry blend of ingredients is fed through a roller compactor and a mill before being tableted in a tablet press.

### Compositions

One aspect of the disclosure is a tablet comprising Compound 1 and/or tautomers thereof or a pharmaceutically acceptable salt or hydrate thereof (referred to herein as "the drug substance" or "DS") and a compressible filler. Any suitable compressible filler may be used. In some embodiments, the compressible filler is a compressible form of microcrystalline cellulose (MCC) or lactose or any combination thereof. For example, the compressible filler is silicified microcrystalline cellulose (SMCC). In another example, the compressible filler is anhydrous lactose. In some embodiments, the compressible filler is an intra-granular compressible filler, which is present as an intra-granular component of the tablet. In some embodiments, the compressible filler may be an extra-granular compressible filler, or a compressible filler in a non-granulated tablet. The intra-granular compressible filler is added to the dry blend prior to granulation. In some embodiments, the weight ratio of the drug substance to the intra-granular compressible filler is greater than 1:1. As discussed in the examples below, tablets with weight ratios of the drug substance to the intra-granular compressible filler (DS:IGCF) of greater than 1:1 (e.g., 3:1 or 6:1) demonstrated improved stability compared to tablets with weight ratios less than 1:1 (e.g., 1:2, 1:3, or 1:4). In some embodiments, the weight ratio of DS:IGCF is greater than about 1.5:1. In some embodiments, the weight ratio of DS:IGCF is less than about 20:1, e.g., less than about 10:1. Examples of ranges of DS:IGCF include from about 1.5:1 to about 20:1, from about 2:1 to about 10:1, and from about 3:1 to about 6:1.

In some embodiments, the intra-granular compressible filler is present in the tablet in an amount of from about 2 wt% to about 15 wt% by weight of the tablet, e.g., from about 3 wt% to about 10 wt% by weight of the tablet. In some embodiments, the intra-granular compressible filler selected from a compressible form of lactose (e.g., anhydrous lactose), a compressible form of MCC (e.g., SMCC), or any combination thereof and is present in an amount of from about 2 wt% to about 15 wt% by weight of the tablet, e.g., from about 3 wt% to about 10 wt% by weight of the tablet. In some embodiments, the intra-granular compressible filler is SMCC. In some embodiments, the intra-granular compressible filler is present in an amount from about 10 wt% to about 25 wt% (e.g., from about 10 wt% to about 20 wt% or from about 13 wt% to about 17 wt%) by weight of the intra-granular component (i.e., excluding the extra-granular component).

In some embodiments, the tablet comprises from about 10 wt% to about 30 wt% of the drug substance. In some embodiments, the intra-granular component comprises from about 40 wt% to about 60 wt% of the drug substance by weight of the intra-granular component.

In certain embodiments tablets of the present disclosure may be produced by a dry granulation process. In another embodiment, the intra-granular component in the tablets may be produced by a direct compression process. The intra-granules produced by the method may have superior flowability and/or be suitable for the mass production (for example, automated mass production using machine) of tablet formulation comprising Compound 1 and/or tautomers thereof or a pharmaceutically acceptable salt or hydrate thereof.

Processes are described in more detail below.

Tablets of the present disclosure may comprise additional excipients, such as low-compressibility fillers, binders, extra-granular fillers, disintegrants, and lubricants.

In some embodiments, the tablet comprises a low-compressibility filler. In some embodiments, the tablet comprises from about 5 wt% to about 20 wt% (e.g., from about 6 wt% to about 17 wt%) of a low-compressibility filler. In some embodiments, the low-compressibility filler is selected from mannitol, a low-compressibility form of lactose, or any combination thereof. In some embodiments, the low-compressibility filler is mannitol. In some embodiments, the low-compressibility filler is present as an intra-granular component of the tablet. For example, the low-compressibility filler may be added to the dry blend prior to granulation (e.g., dry granulation). In some embodiments, the intra-granular component further comprises from about 20 wt% to about 40 wt% (e.g., from about 25 to about 35 wt%) of a low-compressibility filler by weight of the intra-granular component.

In some embodiments, the tablet comprises a binder. In some embodiments, the tablet comprises from about 0.25 wt% to about 2 wt% of a binder (e.g., from about 0.5 wt% to about 1.5 wt%). In some embodiments, the binder is polyvinylpyrrolidone (PVP). In some embodiments, the binder is present as an intra-granular component of the tablet. For example, the binder may be added to the dry blend prior to granulation (e.g., dry granulation). In some embodiments, the intra-granular component further comprises from about 1 wt% to about 3 wt% (e.g., from about 1.5 to about 2.5 wt%) of a binder by weight of the intra-granular component.

In some embodiments, the tablet comprises an extra-granular filler. In some embodiments, the tablet comprises from about 25 wt% to about 80 wt% (e.g., from about 40 wt% to about 75 wt%) of an extra-granular filler. In some embodiments, the extra-granular filler is a compressible filler. In some embodiments, the extra-granular filler is lactose. In some embodiments, the extra-granular filler is anhydrous lactose. The extra-granular filler is present as an extra-granular component of the tablet. For example, the extra-granular filler is added to the dry-granulated blend after granulation. In some embodiments, the extra-granular component further comprises from about 90 wt% to 100 wt% (e.g., from about 90 wt% to about 99 wt%) of an extra-granular filler by weight of the extra-granular component.

In some embodiments, the tablet comprises a disintegrant. In some embodiments, the tablet comprises from about 1 wt% to about 5 wt% (e.g., from about 2 wt% to about 3 wt%) of a disintegrant. In some embodiments, the disintegrant is croscarmellose sodium. The disintegrant may be present as an intra-granular component, an extra-granular component or both.

In some embodiments, the tablet comprises a lubricant. In some embodiments, the tablet comprises from about 0.5 wt% to about 4 wt% (e.g., from about 1 wt% to about 2 wt%) of a lubricant. In some embodiments, the lubricant is magnesium stearate (MgSt). The lubricant may be present as an intra-granular component, an extra-granular component or both.

In some embodiments, where the tablet has an intra-granular component and an extra-granular component, the weight ratio of the intra-granular component to the extra granular component is from about 1:10 to about 3:1 (e.g., from about 1:3 to about 2:1).

In one embodiment, the tablet comprises from about 10 wt% to about 30 wt% of the drug substance, from about 2 wt% to about 15 wt% of intra-granular compressible filler, and from about 5 wt% to about 20 wt% of intra-granular low compressibility filler.

In another embodiment, the tablet comprises from about 10 wt% to about 30 wt% of the drug substance, from about 2 wt% to about 15 wt% of SMCC, and from about 5 wt% to about 20 wt% of intra-granular low compressibility filler.

In another embodiment, the tablet comprises from about 10 wt% to about 30 wt% of the drug substance, from about 3 wt% to about 10 wt% of SMCC, and from about 5 wt% to about 20 wt% of mannitol.

In one embodiment, the tablet comprises from about 10 wt% to about 30 wt% of the drug substance, from about 2 wt% to about 15 wt% of intra-granular compressible filler, from about 5 wt% to about 20 wt% of intra-granular low compressibility filler, and from about 25 wt% to about 80 wt% of extra-granular filler.

In another embodiment, the tablet comprises from about 10 wt% to about 30 wt% of the drug substance, from about 2 wt% to about 15 wt% of SMCC, from about 5 wt% to about 20 wt% of intra-granular low compressibility filler, and from about 25 wt% to about 80 wt% of extra-granular filler.

In another embodiment, the tablet comprises from about 10 wt% to about 30 wt% of the drug substance, from about 3 wt% to about 10 wt% of SMCC, from about 5 wt% to about 20 wt% of mannitol, and from about 25 wt% to about 80 wt% of anhydrous lactose.

In one embodiment, the tablet comprises from about 10 wt% to about 30 wt% of the drug substance, from about 2 wt% to about 15 wt% of intra-granular compressible filler, from about 5 wt% to about 20 wt% of intra-granular low compressibility filler, and from about 0.25 wt% to about 2 wt% of binder.

In another embodiment, the tablet comprises from about 10 wt% to about 30 wt% of the drug substance, from about 2 wt% to about 15 wt% of SMCC, from about 5 wt% to about 20 wt% of intra-granular low compressibility filler, and from about 0.25 wt% to about 2 wt% of binder.

In another embodiment, the tablet comprises from about 10 wt% to about 30 wt% of the drug substance, from about 3 wt% to about 10 wt% of SMCC, from about 5 wt% to about 20 wt% of mannitol, and from about 0.25 wt% to about 2 wt% of PVP.

In one embodiment, the tablet comprises from about 10 wt% to about 30 wt% of the drug substance, from about 2 wt% to about 15 wt% of intra-granular compressible filler, from about 5 wt% to about 20 wt% of intra-granular low compressibility filler, from about 0.25 wt% to about 2 wt% of binder, from about 2 wt% to about 3 wt% of disintegrant, and from about 1 wt% to about 2 wt% of lubricant.

In another embodiment, the tablet comprises from about 10 wt% to about 30 wt% of the drug substance, from about 2 wt% to about 15 wt% of SMCC, from about 5 wt% to about 20 wt% of intra-granular low compressibility filler, from about 0.25 wt% to about 2 wt% of binder, from about 2 wt% to about 3 wt% of disintegrant, and from about 1 wt% to about 2 wt% of lubricant.

In another embodiment, the tablet comprises from about 10 wt% to about 30 wt% of the drug substance, from about 3 wt% to about 10 wt% of SMCC, from about 5 wt% to about 20 wt% of mannitol, from about 0.25 wt% to about 2 wt% of PVP, from about 2 wt% to about 3 wt% of croscarmellose sodium, and from about 1 wt% to about 2 wt% of magnesium stearate.

In one embodiment, the tablet comprises from about 10 wt% to about 30 wt% of the drug substance, from about 2 wt% to about 15 wt% of intra-granular compressible filler, from about 5 wt% to about 20 wt% of intra-granular low compressibility filler, from about 0.25 wt% to about 2 wt% of binder, from about 25 wt% to about 80 wt% of extra-granular filler, from about 2 wt% to about 3 wt% of disintegrant, and from about 1 wt% to about 2 wt% of lubricant.

In another embodiment, the tablet comprises from about 10 wt% to about 30 wt% of the drug substance, from about 2 wt% to about 15 wt% of SMCC, from about 5 wt% to about 20 wt% of intra-granular low compressibility filler, from about 0.25 wt% to about 2 wt% of binder, from about 25 wt% to about 80 wt% of extra-granular filler, from about 2 wt% to about 3 wt% of disintegrant, and from about 1 wt% to about 2 wt% of lubricant.

In another embodiment, the tablet comprises from about 10 wt% to about 30 wt% of the drug substance, from about 3 wt% to about 10 wt% of SMCC, from about 5 wt% to about 20 wt% of mannitol, from about 0.25 wt% to about 2 wt% of PVP, from about 25 wt% to about 80 wt% of anhydrous lactose, from about 2 wt% to about 3 wt% of croscarmellose sodium, and from about 1 wt% to about 2 wt% of magnesium stearate.

In some embodiments, the tablet comprises an intra-granular component and an extra-granular component.

In one embodiment, the intra-granular component comprises (by weight of the intra-granular component) from about 40 wt% to about 60 wt% of the drug substance, and from about 10 wt% to about 25 wt% of compressible filler.

In another embodiment, the intra-granular component comprises (by weight of the intra-granular component) from about 40 wt% to about 60 wt% of the drug substance, and from about 10 wt% to about 25 wt% of SMCC.

In one embodiment, the intra-granular component comprises (by weight of the intra-granular component) from about 40 wt% to about 60 wt% of the drug substance, from about 10 wt% to about 25 wt% of compressible filler, and from about 20 wt% to about 40 wt% of low-compressibility filler.

In another embodiment, the intra-granular component comprises (by weight of the intra-granular component) from about 40 wt% to about 60 wt% of the drug substance, from about 10 wt% to about 25 wt% of SMCC, and from about 20 wt% to about 40 wt% of low-compressibility filler.

In another embodiment, the intra-granular component comprises (by weight of the intra-granular component) from about 40 wt% to about 60 wt% of the drug substance, from about 10 wt% to about 20 wt% of SMCC, and from about 20 wt% to about 40 wt% of mannitol.

In one embodiment, the intra-granular component comprises (by weight of the intra-granular component) from about 40 wt% to about 60 wt% of the drug substance, from about 10 wt% to about 25 wt% of compressible filler, from about 20 wt% to about 40 wt% of low-compressibility filler, from about 1 wt% to about 3 wt% of binder, from about 1 wt% to about 3 wt% of disintegrant, and from about 0.5 wt% to about 2 wt% of lubricant.

In another embodiment, the intra-granular component comprises (by weight of the intra-granular component) from about 40 wt% to about 60 wt% of the drug substance, from about 10 wt% to about 25 wt% of SMCC, from about 20 wt% to about 40 wt% of low-compressibility filler, from about 1 wt% to about 3 wt% of binder, from about 1 wt% to about 3 wt% of disintegrant, and from about 0.5 wt% to about 2 wt% of lubricant.

In another embodiment, the intra-granular component comprises (by weight of the intra-granular component) from about 40 wt% to about 60 wt% of the drug substance, from about 10 wt% to about 20 wt% of SMCC, from about 20 wt% to about 40 wt% of mannitol, from about 1 wt% to about 3 wt% of PVP, from about 1 wt% to about 3 wt% of croscarmellose sodium, and from about 0.5 wt% to about 2 wt% of magnesium stearate.

In some embodiments, the extra-granular component comprises a filler. In some embodiments, the extra-granular component comprises a compressible filler.

In one embodiment, the extra-granular component comprises (by weight of the extra-granular component) from about 90 wt% to 100 wt% of a filler.

In another embodiment, the extra-granular component comprises (by weight of the extra-granular component) from about 90 wt% to 100 wt% of a compressible filler.

In another embodiment, the extra-granular component comprises (by weight of the extra-granular component) from about 90 wt% to 100 wt% of anhydrous lactose.

In one embodiment, the extra-granular component comprises (by weight of the extra-granular component) from about 90 wt% to about 97 wt% of compressible filler, from about 2 wt% to about 5 wt% of disintegrant, and from about 1 wt% to about 3 wt% of lubricant.

In one embodiment, the extra-granular component comprises (by weight of the extra-granular component) from about 90 wt% to about 97 wt% of anhydrous lactose, from about 2 wt% to about 5 wt% of disintegrant, and from about 1 wt% to about 3 wt% of lubricant.

In another embodiment, the extra-granular component comprises (by weight of the extra-granular component) from about 90 wt% to about 97 wt% of anhydrous lactose, from about 2 wt% to about 5 wt% of croscarmellose sodium, and from about 1 wt% to about 3 wt% of magnesium stearate.

In some instances, the weight ratio of the intra-granular component to the extra granular component is from about 1:3 to about 2:1. For example, the weight ratio of the intra-granular component to the extra granular component is about 1:2 or about 1:1.

In another embodiment, the tablet does not comprise an intra-granular component and an extra-granular component. For example, the tablet is made by a method comprising direct compression process, i.e., where the tablet is compressed without granulation. In one embodiment, the tablet comprises the drug substance and a compressible filler. In one embodiment, the compressible filler is SMCC. In another embodiment, the compressible filler is anhydrous lactose. In some embodiments, the tablet comprises additional excipients, such as a binder (e.g., PVP), lubricant (e.g., magnesium stearate) and/or disintegrant (e.g., croscarmellose sodium). The tablet may comprise two or more compressible fillers, e.g., SMCC and anhydrous lactose. In some embodiments, the tablet may further comprise a low-compressibility filler, e.g., mannitol.

In one embodiment, provided herein is a tablet having the ingredients and amounts as shown in Table 1.

**Table 1 10 mg dose; 80 mg tablet**

| | Ingredient | Amount | Wt% of Total | Wt% of Component |
|---|---|---|---|---|
| Intra-granular component | DS | 10 mg | 12.5 wt% | 50 wt% |
| | Mannitol | 6 mg | 7.5 wt% | 30 wt% |
| | SMCC | 3 mg | 3.75 wt% | 15 wt% |
| | PVP | 0.4 mg | 0.5 wt% | 2 wt% |
| | Croscarmellose sodium | 0.4 mg | 0.5 wt% | 2 wt% |
| | MgSt | 0.2 mg | 0.25 wt% | 1 wt% |
| Extra-granular component | Anhydrous lactose | 57.6 mg | 72 wt% | 96 wt% |
| | Croscarmellose sodium | 1.6 mg | 2 wt% | 2.67 wt% |
| | MgSt | 0.8 mg | 1 wt% | 1.33 wt% |

In one embodiment, provided herein is a tablet having the ingredients and amounts as shown in Table 2.

**Table 2 25 mg dose; 100 mg tablet**

| | Ingredient | Amount | Wt% of Total | Wt% of Component |
|---|---|---|---|---|
| Intra-granular component | DS | 25 mg | 25 wt% | 50 wt% |
| | Mannitol | 15 mg | 15 wt% | 30 wt% |
| | SMCC | 7.5 mg | 7.5 wt% | 15 wt% |
| | PVP | 1 mg | 1 wt% | 2 wt% |
| | Croscarmellose sodium | 1 mg | 1 wt% | 2 wt% |
| | MgSt | 0.5 mg | 0.5 wt% | 1 wt% |
| Extra-granular component | Anhydrous lactose | 47 mg | 47 wt% | 94 wt% |
| | Croscarmellose sodium | 2 mg | 2 wt% | 4 wt% |
| | MgSt | 1 mg | 1 wt% | 2 wt% |

In one embodiment, provided herein is a tablet having the ingredients and amounts as shown in Table 3.

**Table 3 80 mg dose; 320 mg tablet**

| | Ingredient | Amount | Wt% of Total | Wt% of Component |
|---|---|---|---|---|
| Intra-granular component | DS | 80 mg | 25 wt% | 50 wt% |
| | Mannitol | 48 mg | 15 wt% | 30 wt% |
| | SMCC | 24 mg | 7.5 wt% | 15 wt% |
| | PVP | 3.2 mg | 1 wt% | 2 wt% |
| | Croscarmellose sodium | 3.2 mg | 1 wt% | 2 wt% |
| | MgSt | 1.6 mg | 0.5 wt% | 1 wt% |
| Extra-granular component | Anhydrous lactose | 150.4 mg | 47 wt% | 94 wt% |
| | Croscarmellose sodium | 6.4 mg | 2 wt% | 4 wt% |
| | MgSt | 3.2 mg | 1 wt% | 2 wt% |

In one embodiment, provided herein is a tablet including the ingredients and amounts as shown in Table 4 as intra-granular component.

**Table 4 100 mg dose**

| | Ingredient | Amount |
|---|---|---|
| Intra-granular component | DS | 100 mg |
| | Mannitol | 60mg |
| | SMCC | 30 mg |
| | PVP | 4 mg |
| | Croscarmellose sodium | 4 mg |
| | MgSt | 2 mg |

The tablet of the present disclosure may further contain one or more additives conventionally used in the field of pharmaceutical preparation. Examples of the conventional additives include, but not limited to, colorants, flavorings, preservatives, sweeteners, glidants, coating materials. Unless particularly indicated, these additives are used in an amount conventionally employed in the field of pharmaceutical preparation. In some embodiments, the colorants include food colors such as Food Color Yellow No. 5, Food Color Red No. 2, Food Color Blue No. 2, food lake colors, red ferric oxide (diiron trioxide), yellow ferric oxide, OPADRY Red (Trade name), OPADRY Yellow (Trade name), or mixture thereof.

In some embodiments, the flavorings is selected from mint or vanilla, or a combination thereof.

In some embodiments, the preservatives is an antioxidant. In certain embodiments the antioxidant is selected from vitamin A, C, and E, or a combination thereof.

In some embodiments, the sweeteners is selected from sugar and artificial sweeteners.

In some embodiments, the glidant is selected from talc, magnesium carbonate, or a combination thereof.

In some embodiments, the coating materials are selected from light shielding agents such as titanium dioxide.
Unless particularly indicated, these additives are used in an amount conventionally employed in the field of pharmaceutical preparation.

The drug substance of the present disclosure is Compound 1 and/or tautomers thereof or a pharmaceutically acceptable salt or hydrate thereof. Compound 1 has the following structure:

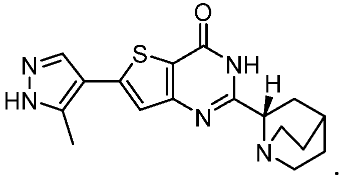

The chemical name for Compound 1 is 2-[(2S)-1-azabicyclo[2.2.2]oct-2-yl]-6-(3-methyl-1H-pyrazol-4-yl)thieno[3,2-d]pyrimidin-4(3H)-one. Compound 1 is a Cdc7 kinase inhibitor.

Tautomers of Compound 1 or a pharmaceutically acceptable salt or hydrate of Compound 1 are/is also encompassed by the present disclosure. When Compound 1 has a tautomer, each isomer is also encompassed in the present disclosure.

As used herein the phrase "Compound 1 and/or tautomers thereof" is understood to mean Compound 1 and all of its tautomeric forms. As a non-limiting example, tautomerization may occur in the pyrazole and pyrimidine groups of Compound 1. Specific examples of tautomerization that may occur in Compound 1 include:

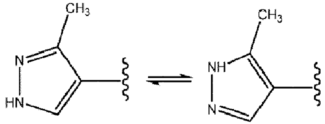

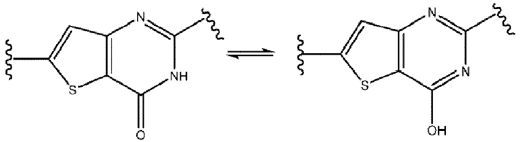

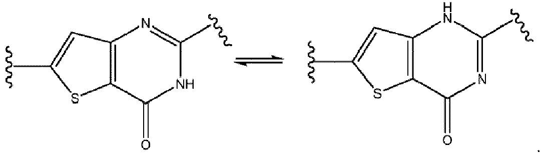

Compound 1 and/or tautomers thereof can be used in the form of a pharmaceutically acceptable salt. Examples of the pharmaceutically acceptable salt include salts with inorganic bases, salts with organic bases, salts with inorganic acids, salts with organic acids, and salts with basic or acidic amino acids.

Compound 1 and/or tautomers thereof may be a hydrate (e.g., hemihydrate), a non-hydrate, a solvate or a non-solvate, all of which are encompassed in the present disclosure. In some embodiments, Compound 1 and/or tautomers thereof is a hemihydrate.

Compound 1 and/or tautomers thereof or a pharmaceutically acceptable salt or hydrate thereof or a crystal form thereof can be obtained according to the production methods described in PCT Publication No. WO 2011/102399, U.S. Patent No. 8,722,660, U.S. Patent No. 8,921,354, U.S. Patent No. 8,933,069, and U.S. Patent Publication No. US 2015/158882.

Compound 1 and/or tautomers thereof or a pharmaceutically acceptable salt or hydrate thereof may be in the form of a crystal (e.g., crystalline form A and crystalline form I), and the crystal form of the crystal may be single or plural, both of which are encompassed in Compound 1. The crystal may be of a form, and can be produced by a method, described in PCT publication no. WO 2017/172565, published October 5, 2017. In some embodiments, the Compound 1 and/or tautomers thereof or a pharmaceutically acceptable salt or hydrate thereof may be in the form of Crystalline Form I as described in WO 2017/172565. In some embodiments, the Compound 1 and/or tautomers thereof or a pharmaceutically acceptable salt or hydrate thereof is a crystalline form of Compound 1 hemihydrate (i.e., 2-[(2S)-1-azabicyclo[2.2.2]oct-2-yl]-6-(3-methyl-lH-pyrazol-4-yl)thieno[3,2-d]pyrimidin-4(3H)-one hemihydrate). For example, the Compound 1 and/or tautomers thereof or a pharmaceutically acceptable salt or hydrate thereof may be Crystalline Form I of Compound 1 hemihydrate.

Compound 1 and/or tautomers thereof or a pharmaceutically acceptable salt or hydrate thereof is stable and has low toxicity, and can be used safely. While the daily dose varies depending on the condition and body weight of the patient, and administration route, in the case of, for example, Compound 1 and/or tautomers thereof or a pharmaceutically acceptable salt or hydrate thereof may be administered orally in the form of a medicament described herein to a patient for treatment.

In some embodiments, the pharmaceutical composition of the present disclosure comprises a dose strength of Compound 1 and/or tautomers thereof or a pharmaceutically acceptable salt or hydrate thereof ranging from 5 to 200 mg. For example, in some embodiments, a medicament comprises a dose strength of 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, or 200 mg of Compound 1 and/or tautomers thereof or a pharmaceutically acceptable salt or hydrate thereof.

### Methods

Another aspect of the present disclosure is a method of making a tablet comprising Compound 1 and/or tautomers thereof or a pharmaceutically acceptable salt or hydrate thereof

In some embodiments, the method comprises a dry-granulation method. In some embodiments, the method comprises mixing the drug substance and a compressible filler to form a dry blend, granulating the dry blend to form a dry-granulated blend, and compressing the dry-granulated blend to form the tablet. The tablet formed by the present methods may comprise the ingredients, weight ratios, and amounts as described herein, i.e., by providing the desired ingredients in the desired amounts.

In some embodiments, the method further comprises mixing the drug substance and compressible filler with one or more additional excipients. For example, one or more intra-granular excipients may be added to the dry blend to be dry-granulated with the drug substance and the compressible filler. Alternatively, or in conjunction, one or more extra-granular excipients may be added after dry-granulation of the drug substance, compressible filler, and optional excipients. As described above, non-limiting examples of the one or more excipients include lubricants (e.g., magnesium stearate), low-compressibility fillers (e.g., mannitol), extra-granular fillers (e.g., anhydrous lactose), binders (e.g., PVP), and disintegrants (e.g., croscarmellose sodium).

In some embodiments, granulating comprises compacting the dry blend to form a compacted dry-granulated blend and milling the compacted blend to form a milled dry-granulated blend. The milling step may produce a milled dry-granulated blend with a mean particle size from about 120 microns to about 180 microns. In some embodiments, the method further comprises compressing the milled dry-granulated blend to form the tablet. In some embodiments, the method further comprises adding an extra-granular component to the dry-granulated blend, (e.g., the milled dry-granulated blend) prior to tableting. The extra-granular component may comprise lactose (e.g., anhydrous lactose).

In some embodiments, lubricant and disintegrant are premixed before mixing with other excipients. In some embodiments, premixed lubricant and disintegrant are then mixed with the drug substance, compressible filler, low-compressibility filler, and binder. The mixture may then be compacted by a roller compactor to form a coarse ribbon granule followed by a milling step to form a milled granule. The milled granule may be mixed with the extra-granular components (e.g., extra-granular filler, disintegrant and lubricant), which may then be tableted.

Preblending of lubricant and disintegrant may be performed in a diffusion mixer for about 1-10 minutes at a speed of about 20-40 rpm. Intra-granular blending may be performed in a diffusion mixer for about 10 to 20 minutes at about 20-40 rpm. Roller compaction may be performed by a roller compactor at a hydraulic pressure of about 4-8 (e.g., 5-6) MPa, with a max CP of about 40-80 (e.g., 50-60) MPa, a screw speed of about 30-50 rpm, and a roller speed of about 5-20 rpm. Milling may be performed in a co-mill unit with a 0.5 to 1 (e.g., 0.7-0.9) mm screen at a speed of about 1800-2200 rpm. Post-granulation blending with the extra-granular component may be in a diffusion mixer for about 5-20 min at a speed of about 20-40 rpm. Tableting may be performed with a tablet press at a target compression pressure of from about 150 to about 250 (e.g., about 200) MPa.

As particle size of intra-granular particle, 100-250 (e.g., 120-180) micron is preferable in terms of higher blend conformity and/or higher content uniformity.

In another embodiment, the tablet is made by a direct compression method. For example, the method comprises mixing the drug substance and a compressible filler to form a dry blend and compressing the dry blend to form a tablet. In some instances, the method further comprises milling the dry blend to form a milled dry blend, and compressing the milled dry blend to form the tablet. In a direct compression method, the blend is not granulated, e.g., no roller compactor is used to form granules.

A conventional protective coating layer may also be applied to tablets by conventional coating techniques such as pan coating or fluid bed coating.

In some embodiments, the coating is with colorant.

In some embodiments, the coating is without colorant. In some embodiments, the coating bases are sugar coating base (e.g., sucrose) and aqueous film coating base. In some embodiments, the aqueous film coating base is a cellulose polymer selected from hydroxypropylcellulose, hydroxypropylmethylcellulose, hydroxyethylcellulose, and methylhydroxyethylcellulose. In some embodiments, the aqueous film coating base is a synthetic polymer selected from polyvinyl acetal diethylaminoacetate, aminoalkylmethacrylate copolymer E, and polyvinylpyrrolidone; and polysaccharides such as pullulan.

### EXAMPLES

In the following example, prototypes F1-F12 are not presently claimed and are provided for illustrative purposes.

### Reference Example 1: Excipient Compatibility Study

Tablets with different formulation composites were manufactured to determine which tablets would provide adequate stability. The study evaluated 2 types of compressible filler: anhydrous lactose and silicified microcrystalline cellulose (SMCC), and several different weight ratios between the drug substance (DS) and the compressible filler. The tablet with higher weight ratio of drug substance to compressible filler demonstrated better stability. The two compressible fillers showed similar stability.

The formulation compositions of the tablet prototypes are described in Table 5. The excipients and the drug substance were weighed and blended in glass vial using a vibrator, and compressed using a Carver hydraulic press with a 3/8" standard round flat tooling at 6-8kN. The tablets were stored at the accelerated conditions of 60°C/75% RH.

**Table 5 Composition of Prototype Tablets for Formulation Screening**

| **Component** | **Amount (mg)** | | | | | | | | |
|---|---|---|---|---|---|---|---|---|---|
| | **F1** | **F2** | **F3** | **F4** | **F5** | **F6** | **F7** | **F8** | **F9** |
| DS (micronized) | 50 | 50 | 50 | 50 | 50 | 50 | 150 | 150 | 150 |
| SMCC (Prosolv SMCC 50) | 173 | 173 | 173 | 173 | - | 200 | 50 | 25 | - |
| MCC (Ceolus 101) | - | - | - | - | 94 | - | - | - | - |
| Lactose (SuperTab 24AN) | - | - | - | - | - | - | - | - | 50 |
| Mannitol (Pearlitol 100SD) | - | - | - | - | 94 | - | 85 | 110 | 85 |
| Ac-Di-Sol | 15 | 15 | 15 | 15 | - | - | 6 | 6 | 6 |
| HPC-L | - | - | - | - | 9 | - | - | - | - |
| PVP (Plasdone K-30) | 9 | - | 9 | - | - | - | 6 | 6 | 6 |
| HPMC (HPMCP HP-50) | - | 9 | - | 9 | - | - | - | - | - |
| Talc | 3 | 3 | - | - | - | - | 3 | 3 | 3 |
| MgSt | - | - | - | - | 3 | - | - | - | - |
| Fumed silica (Aerosil 200) | - | - | 3 | 3 | - | - | - | - | - |
| Total Weight (mg) | 250 | 250 | 250 | 250 | 250 | 250 | 300 | 300 | 300 |

The results from the compatibility study are shown in Table 6.

**Table 6 Stability Results for Tablet Formulation Screening**

| **Proto-type** | **Ratio of DS: compressible filler** | **Initial** | | **60°C/75% RH open** | | **60°C/75% RH open** | |
|---|---|---|---|---|---|---|---|
| | | | | **1 week** | | **1 month** | |
| | | **Impurity (%)** | **Enantiomer (%)** | **Impurity (%)** | **Enantiomer (%)** | **Impurity (%)** | **Enantiomer (%)** |
| F1 | DS : SMCC = 0.29:1 | 0.00 | 1.64 | 0.06 | 2.84 | ND | ND |
| F2 | DS : SMCC = 0.29:1 | 0.00 | ND | 0.11 | 2.73 | ND | ND |
| F3 | DS : SMCC = 0.29:1 | 0.00 | ND | 0.07 | 3.02 | ND | ND |
| F4 | DS : SMCC = 0.29:1 | 0.00 | ND | 0.12 | 3.07 | ND | ND |
| F5 | DS : MCC = 0.53:1 | 0.00 | ND | 000 | 2.35 | ND | ND |
| F6 | DS : SMCC = 0.25:1 | 0.00 | ND | 0.00 | 2.85 | ND | ND |
| F7 | DS : SMCC = 3:1 | 0.00 | 1.61 | 000 | 1.77 | 0.00 | 200 |
| F8 | DS : SMCC = 6:1 | 0.00 | 1.62 | 000 | 1.73 | 000 | 1.92 |
| F9 | DS : lactose = 3:1 | 0.00 | 1.61 | 000 | 1.77 | 0.00 | 1.87 |
| DS = drug substance; RH = relative humidity; ND = not determined. | | | | | | | |

When weight ratio between DS and compressible filler was below 1:1 in the tablet (prototypes F1-F6), tablet at 1 week time point showed more than 0.7% increase of enantiomer in the accelerated condition, and was not further tested at 1 month. When the ratio was changed to 3:1 or 6:1 in prototypes F7-F9, tablet with either SMCC or anhydrous lactose as the compressible filler showed less than 0.2% increase of enantiomer at 1 week. Further tests showed that the increase was less than 0.4% at 1 month. Based on the results, anhydrous lactose and SMCC were both selected for further development at a weight ratio of DS:CF of about 3:1.

### Reference Example 2: Manufacturing Process Development

To prevent micronizing of the drug substance resulting in cohesive, sticky powder, the flowability and compressibility of prototype formulations was quantitatively studied. Manufacturability of direct compression process versus dry granulation process on the selected prototypes was evaluated. By using a compaction simulator (Huxley Bertram ESH, Cambridge UK), the prototype blends were compressed into tablets directly, or compressed into ribbons, comilled, and finally compressed into tablets. It was determined that the combination of dry granulation process and the blend of SMCC formulation improved powder flow, eliminated sticky issue, reduced variability of tablet weight, and therefore provided the best manufacturability. In this formulation, mannitol was selected as intra-granular low-compressibility filler, SMCC were selected as intra-granular compressible filler, and PVP was chosen to be intra-granular binder. Anhydrous lactose (SuperTab 24AN) was selected as extra-granular filler. Ac-Di-Sol (croscarmellose sodium) was chosen as the disintegrant, and MgSt (magnesium stearate) the lubricant in both intra- and extra-granular fractions of the formulation. Based on the compaction simulation results on the roller compaction unit operation and the tablet compression unit operation, the operating condition for small scale manufacture was determined. The compositions of prototype tablets in the study are shown in Table 7.

**Table 7 Composition of Prototype Tablets for Manufacturability Study**

| **Component** | **Amount (% w/w)** | | |
|---|---|---|---|
| | **F10** | **F11** | **F12** |
| DS (micronized) | 50 | 50 | 50 |
| SMCC (Prosolv SMCC 50) | 15 | 15 | - |
| Lactose (SuperTab 24AN) | - | - | 15 |
| Mannitol (Pearlitol 100SD) | 30 | 30 | 30 |
| Ac-Di-Sol | 2 | 2 | 2 |
| PVP (Plasdone K-30) | 2 | 2 | 2 |
| MgSt | - | 1 | 1 |
| Fumed silica (Aerosil 200) | 1 | - | - |
| Powder True Density (g/cc) | ND | 1.46 | 1.45 |
| Tooling shape | 3/8" standard round flat | | |
| Total weight (mg) | 220 | | |

Three different techniques can be utilized for the tablet drug product (DP) manufacture: direct compression (DC), dry granulation (DG), or wet granulation (WG). Due to the potential concern of enantiomer increase under high humidity or high temperature conditions, WG was excluded. In this study, the Huxley Bertram ESH tablet compaction simulator (Cambridge UK) was applied to simulate the DC and the DG manufacturing processes. Active blend was either directly compressed or was dry-granulated first before tablet compression using the simulator.

For the simulation of the DG process, compression pressure was adjusted to produce ribbon of different porosity. The flowability and compressibility of either preblend or milled ribbon was measured to evaluate the manufacturability of each formulation on the DC and the DG processes. The peak force value of compression and the vertical projection area of tooling were used to calculate the maximum compression pressure (CP). Powder flowability (i.e., flow factor (FFc)) was measured using the FT4 powder rheometer with the characterization method of 3 kPa pre-consolidation and 10 ml filling volume (Freeman Technology, Tewkesbury UK). This method was applied because it was representative of powder flow scenarios in most pharmaceutical manufacturing processes. The true density of powder was measured using the AccuPyc 1340 pycnometer (Micrometrics, Norcross GA USA) to calculate porosity of the ribbon.

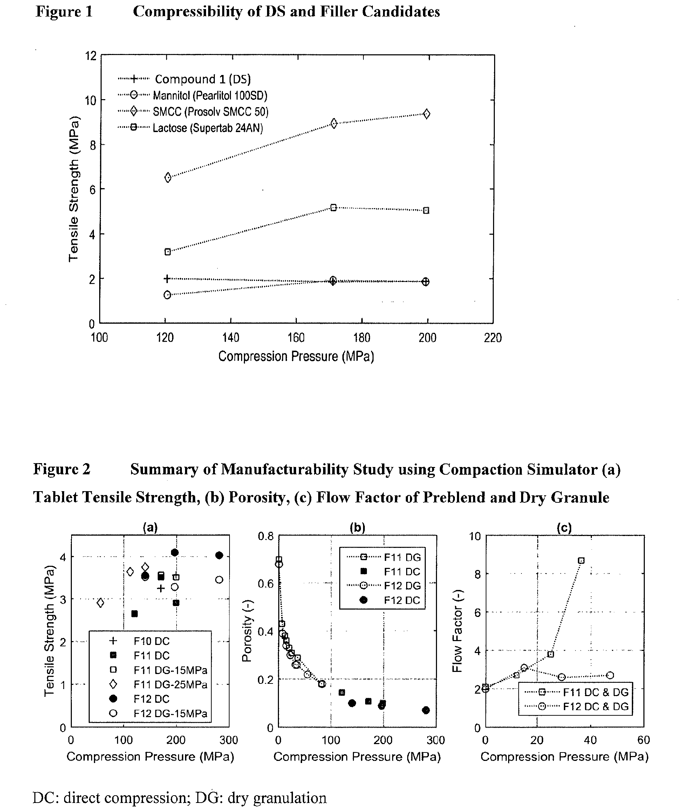

Before performing direct compression of formulation prototypes, the compressibility of the drug substance (DS) and single fillers were tested. The results are shown in Figure 1. The results indicate that SMCC and anhydrous lactose have much higher compactability than drug substance and mannitol, suggesting that SMCC or anhydrous lactose could be used as compressible filler in the tablet manufacture process.

Results of this study are summarized in Table 8. Capping was observed in the DC study of F10 prototype at different compression pressures until MgSt was spread on the tooling head. It indicated the poor manufacturability of the F10 formulation when fumed silica was used as the flow aid. Therefore, prototype F10 was not pursued further.

**Table 8 Summary of Manufacturability Study using Compaction Simulator**

| **Prototype** | **Mfg Method** | **Dry Granulation** | | | **Tablet Compression** | | | |
|---|---|---|---|---|---|---|---|---|
| | | **CP (MPa)** | **Porosity (-)** | **FFc (-)** | **CP (MPa)** | **Poros ity (-)** | **Tensile Strength (MPa)** | **Disintegration (min)** |
| F10 | DC | - | - | - | 121 | - | 1.24 | - |
| | | | | | 171 | - | 3.25 | - |
| | | | | | 199 | - | 3.56 | - |
| F11 | DG | 0 | 0.70 | 2.07 | - | - | - | - |
| | | 7 | 0.43 | - | - | - | - | - |
| | | 12 | 0.38 | 2.70 | - | - | - | - |
| | | 15 | 0.36 | - | 121 | - | 2.66 | - |
| | | | | | 171 | - | 3.56 | - |
| | | | | | 199 | - | 3.51 | - |
| | | 20 | 0.33 | - | - | - | - | - |
| | | 25 | 0.31 | 3.78 | 56 | - | 2.91 | - |
| | | | | | 112 | - | 3.64 | - |
| | | | | | 140 | - | 3.75 | <3 |
| | | 36 | 0.29 | 8.68 | - | - | - | - |
| | | 82 | 0.18 | - | - | - | - | - |
| | DC | - | - | - | 121 | 0.14 | 2.65 | - |
| | | | | | 171 | 0.11 | 3.51 | - |
| | | | | | 199 | 0.10 | 2.91 | <3 |
| F12 | DG | 0 | 0.68 | 2.01 | - | - | - | - |
| | | 8 | 0.39 | - | - | - | - | - |
| | | 15 | 0.33 | 3.06 | 140 | - | 3.52 | - |
| | | | | | 197 | - | 3.28 | - |
| | | | | | 281 | - | 3.45 | - |
| | | 22 | 0.30 | - | - | - | - | - |
| | | 29 | 0.26 | 2.61 | - | - | - | - |
| | | 47 | 0.26 | 2.65 | - | - | - | - |
| | | 55 | 0.22 | - | - | - | - | - |
| | | 83 | 0.18 | - | - | - | - | - |
| | DC | - | - | - | 140 | 0.10 | 3.55 | - |
| | | | | | 197 | 0.09 | 4.10 | - |
| | | | | | 281 | 0.07 | 4.03 | - |

During the study of DC for F11 and F12, large variability was observed on the tablet weight during continuous simulations of direct compression. Comparatively, milled granule produced through the DG process showed superior flowability. Based on the observations on powder flowability and sticking issue, it was found that dry granulation process was more preferable as a step included for tablet manufacturing.

### Example 3: Tablet Compactability Study

The tablet compactability information in both DC and DG manufacturing processes is summarized in Figure 2a. The ribbon or tablet porosity of prototypes F11 and F12 is summarized in Figure 2b. The flow factor of preblend and milled granule for prototypes F11 and F12 is summarized in Figure 2c. It was found that the tensile strength of tablets of all three prototypes were between 2.5 and 4.5 MPa in the compression pressure range 150-300MPa, indicating good compressibility of the formulation prototypes. For both F11 and F12, material porosity dropped faster in the compression pressure range 0-100 MPa than in the range 100-300 MPa. On the other hand, although DG improved F11 flow factor to above 4 (easy flow) when dry granulation compression pressure is above 25MPa, flow factor results of F12 DG milled granules were always below 4 (cf. flow factor of Compound 1 is about 2.3). As a result, it was found that SMCC was more suitable as the compressible filler compared with lactose. Further, through comparison of Flow Factors in F11 of Figure 2c between (i) preblend manufactured by DC (of which Flow Factor shown by point in Compression Pressure is 0) and (ii) Dry Granule manufactured by DG (of which Flow Factor shown by point in Compression Pressure is larger than 0), it was found that a dry granulation process was more preferable as a step for tablet manufacturing in terms of flowability.

### Example 4: Tablet formulation

The following tablet formulations shown in Table 9 were manufactured.

**Table 9 Tablet Formulation**

| **Ingredient** | | **Dose (mg)** | | | **Function** | **Grade** |
|---|---|---|---|---|---|---|
| | | **10** | **25** | **80** | | |
| Intra-granular | DS (Form I) | 10.0 | 25.0 | 80.0 | Active | NA |
| | Mannitol | 6.0 | 15.0 | 48.0 | Filler | Pearlitol 100SD |
| | SMCC | 3.0 | 7.5 | 24.0 | Filler | Prosolv SMCC 50 |
| | PVP | 0.4 | 1.0 | 3.2 | Binder | Plasdone K-30 |
| | Ac-Di-Sol | 0.4 | 1.0 | 3.2 | Disintegrant | SD-711 |
| | MgSt | 0.2 | 0.5 | 1.6 | Lubricant | NA |
| Extra-granular | Lactose | 57.6 | 47.0 | 150.4 | Filler | SuperTab 24AN |
| | Ac-Di-Sol | 1.6 | 2.0 | 6.4 | Disintegrant | SD-711 |
| | MgSt | 0.8 | 1.0 | 3.2 | Lubricant | NA |
| Total (mg) | | 80.0 | 100.0 | 320.0 | - | - |

The manufacturing process is summarized in Table 10. In this case, MgSt and Ac-Di-Sol were mixed in the blender at the beginning before addition of other ingredients. The purpose was to further mitigate the DS sticking issue during preblending.

**Table 10 Tablet Formulation**

| **Process Step** | **Material** |
|---|---|
| **Preblending** | |
| Time: 5 min | Ac-Di-Sol |
| | MgSt |
| Blending | DS API |
| Time: 15 min | Mannitol |
| | SMCC |
| | PVP |
| **Roller Compaction** | Preblend |
| **Milling** | Coarse ribbon granule |
| **Final Blending** | Milled granule |
| Blending time: 10 min | Lactose |
| Lubrication time: 5 min | Ac-Di-Sol |
| | MgSt |
| **Tableting** | Final blend |
| Target compression pressure: 200 MPa | |

Tablets were packaged as demo stability samples with 30 tablets in a 60 cc HDPE bottle and induction sealing, with 2 grams of silica gel. The up to 3-month stability data is available and is shown in Table 11. No issue was observed in the stability data.

**Table 11 Up to 3 Months Stability Data**

| Dose (mg) | | 10 mg | | | 25 mg | | | 80 mg | | |
|---|---|---|---|---|---|---|---|---|---|---|
| Condition | Test | Initial | 1M | 3M | Initial | 1M | 3M | Initial | 1M | 3M |
| 5°C | Assay (%) | 102.5 | 98.5 | ND | 99.4 | 98.3 | ND | 99.7 | 97.7 | ND |
| | Impurities (%) | ND | ND | ND | ND | ND | ND | ND | ND | ND |
| | Chiral (%) | 0.71 | 0.71 | ND | 0.71 | 0.71 | ND | 0.71 | 0.71 | ND |
| | Moisture (%) | 1.45 | 1.18 | ND | 1.88 | 1.51 | ND | 1.74 | 1.37 | ND |
| | Dissolution (%) | | | | | | | | | |
| | 15 min | 92.4 | 94.0 | ND | 93.2 | 93.4 | ND | 92.6 | 92.3 | ND |
| | 30 min | 102.5 | 103.4 | ND | 100.1 | 101.7 | ND | 100.4 | 100.6 | ND |
| | 45 min | 102.5 | 103.4 | ND | 99.9 | 101.6 | ND | 99.9 | 100.7 | ND |
| | 60 min | 102.4 | 103.5 | ND | 99.9 | 101.6 | ND | 99.8 | 100.6 | ND |
| | 75 min | 102.4 | 103.5 | ND | 99.9 | 101.6 | ND | 100.6 | 100.6 | ND |
| 25°C | Assay (%) | 102.5 | 100.7 | 97.9 | 99.4 | 98.8 | 100.9 | 99.7 | 96.2 | 100.4 |
| 60% RH | Impurities (%) | ND | ND | ND | ND | ND | ND | ND | ND | ND |
| | Chiral (%) | 0.71 | 0.71 | 0.70 | 0.71 | 0.71 | 0.70 | 0.71 | 0.71 | 0.70 |
| | Moisture (%) | 1.45 | 1.18 | 1.03 | 1.88 | 1.51 | 1.33 | 1.74 | 1.48 | 1.33 |
| | Dissolution (%) | | | | | | | | | |
| | 15 min | 92.4 | 92.6 | 92.3 | 93.2 | 92.6 | 93.2 | 92.6 | 92.9 | 93.0 |
| | 30 min | 102.5 | 102.3 | 102.1 | 100.1 | 101.4 | 101.2 | 100.4 | 100.5 | 99.9 |
| | 45 min | 102.5 | 102.2 | 102.4 | 99.9 | 101.4 | 101.3 | 99.9 | 100.7 | 99.8 |
| | 60 min | 102.4 | 102.2 | 102.1 | 99.9 | 101.3 | 101.3 | 99.8 | 100.6 | 100.2 |
| | 75 min | 102.4 | 102.2 | 102.1 | 99.9 | 101.2 | 101.2 | 100.6 | 100.5 | 100.3 |
| 40°C | Assay (%) | 102.5 | 98.3 | 100.0 | 99.4 | 96.9 | 100.1 | 99.7 | 97.3 | 100.6 |
| 75% RH | Impurities (%) | ND | ND | ND | ND | ND | ND | ND | ND | ND |
| | Chiral (%) | 0.71 | 0.71 | 0.72 | 0.71 | 0.71 | 0.71 | 0.71 | 0.71 | 0.71 |
| | Moisture (%) | 1.45 | 1.12 | 1.07 | 1.88 | 1.49 | 1.40 | 1.74 | 1.47 | 1.40 |

| | Dissolution (%) | | | | | | | | | |
|---|---|---|---|---|---|---|---|---|---|---|
| | 15 min | 92.4 | 93.1 | 93.4 | 93.2 | 92.5 | 92.3 | 92.6 | 92.6 | 92.7 |
| | 30 min | 102.5 | 102.9 | 100.9 | 100.1 | 100.7 | 100.8 | 100.4 | 100.1 | 100.9 |
| | 45 min | 102.5 | 102.9 | 100.9 | 99.9 | 100.8 | 100.8 | 99.9 | 100.2 | 101.2 |
| | 60 min | 102.4 | 102.9 | 100.9 | 99.9 | 100.7 | 100.9 | 99.8 | 100.2 | 100.4 |
| | 75 min | 102.4 | 102.9 | 101.0 | 99.9 | 100.7 | 100.9 | 100.6 | 100.1 | 100.1 |

## Claims

1. A tablet comprising:
from about 10 wt% to about 30 wt% of Compound 1 and/or tautomers thereof or a pharmaceutically acceptable salt or hydrate thereof 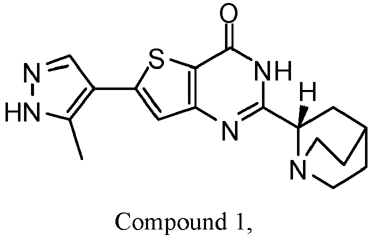
from about 3 wt% to about 10 wt% of silicified microcrystalline cellulose,
from about 5 wt% to about 20 wt% of mannitol,
from about 0.25 wt% to about 2 wt% of polyvinylpyrrolidone,
from about 25 wt% to about 80 wt% of anhydrous lactose,
from about 2 wt% to about 3 wt% of croscarmellose sodium, and
from about 1 wt% to about 2 wt% of magnesium stearate.

2. The tablet of claim 1, wherein the tablet is produced by a method comprising dry granulation process.

3. A tablet comprising an intra-granular component and an extra-granular component, wherein the intra-granular component comprises:
from about 40 wt% to about 60 wt% of Compound 1 and/or tautomers thereof or a pharmaceutically acceptable salt or hydrate thereof 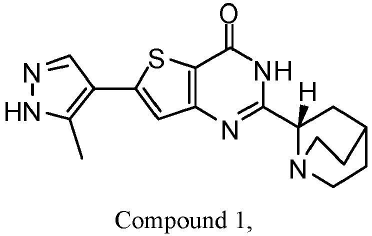
from about 10 wt% to about 20 wt% of silicified microcrystalline cellulose,
from about 20 wt% to about 40 wt% of mannitol,
from about 1 wt% to about 3 wt% of polyvinylpyrrolidone,
from about 1 wt% to about 3 wt% of croscarmellose sodium, and
from about 0.5 wt% to about 2 wt% of magnesium stearate; and
wherein the extra-granular component comprises:
from about 90 wt% to about 98 wt% of anhydrous lactose,
from about 2 wt% to about 5 wt% of croscarmellose sodium, and
from about 1 wt% to about 3 wt% of magnesium stearate.

4. The tablet of claim 3, wherein the weight ratio of the intra-granular component to the extra granular component is from about 1:3 to about 2:1.

5. The tablet of claim 3 or 4, wherein the tablet is produced by a method comprising dry granulation process.

6. The tablet of claim 1, comprising:
| | |
|---|---|
| Compound 1 and/or tautomers thereof or a pharmaceutically acceptable salt or hydrate thereof | 12.5 wt% |
| 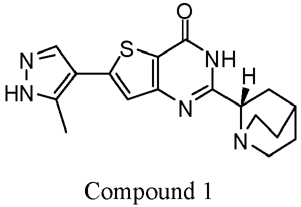 | |
| Mannitol | 7.5 wt% |
| Silicified microcrystalline cellulose | 3.75 wt% |
| Polyvinylpyrrolidone | 0.5 wt% |
| Croscarmellose sodium | 2.5 wt% |
| Magnesium stearate | 1.25 wt% |
| Anhydrous lactose | 72 wt% |

7. The tablet of claim 1, comprising:
| | |
|---|---|
| Compound 1 and/or tautomers thereof or a pharmaceutically acceptable salt or hydrate thereof | 25 wt% |
| 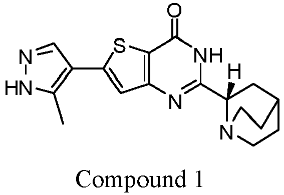 | |
| Mannitol | 15 wt% |
| Silicified microcrystalline cellulose | 7.5 wt% |
| Polyvinylpyrrolidone | 1 wt% |
| Croscarmellose sodium | 3 wt% |
| Magnesium stearate | 1.5 wt% |
| Anhydrous lactose | 47 wt% |

## Patentansprüche

1. Tablette, umfassend:
von etwa 10 Gew.-% bis etwa 30 Gew.-% von Verbindung 1 und/oder Tautomere davon oder ein pharmazeutisch unbedenkliches Salz oder Hydrat davon 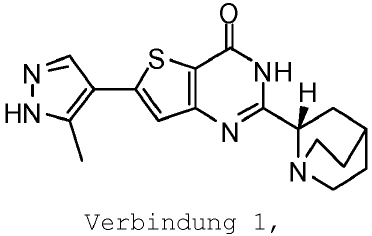
von etwa 3 Gew.-% bis etwa 10 Gew.-% silicifizierte mikrokristalline Cellulose,
von etwa 5 Gew.-% bis etwa 20 Gew.-% Mannit,
von etwa 0,25 Gew.-% bis etwa 2 Gew.-% Polyvinylpyrrolidon,
von etwa 25 Gew.-% bis etwa 80 Gew.-% wasserfreie Lactose,
von etwa 2 Gew.-% bis etwa 3 Gew.-% Croscarmellose-Natrium und
von etwa 1 Gew.-% bis etwa 2 Gew.-% Magnesiumstearat.

2. Tablette nach Anspruch 1, wobei die Tablette durch ein Verfahren hergestellt ist, das einen Trockengranulationsprozess umfasst.

3. Tablette, umfassend eine intragranuläre Komponente und eine extragranuläre Komponente, wobei die intra-granuläre Komponente Folgendes umfasst:
von etwa 40 Gew.-% bis etwa 60 Gew.-% von Verbindung 1 und/oder Tautomere davon oder ein pharmazeutisch unbedenkliches Salz oder Hydrat davon 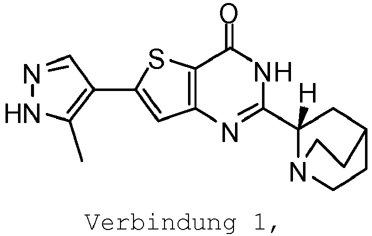
von etwa 10 Gew.-% bis etwa 20 Gew.-% silicifizierte mikrokristalline Cellulose,
von etwa 20 Gew.-% bis etwa 40 Gew.-% Mannit,
von etwa 1 Gew.-% bis etwa 3 Gew.-% Polyvinylpyrrolidon,
von etwa 1 Gew.-% bis etwa 3 Gew.-% Croscarmellose-Natrium und
von etwa 0,5 Gew.-% bis etwa 2 Gew.-% Magnesiumstearat; und
wobei die extragranuläre Komponente Folgendes umfasst:
von etwa 90 Gew.-% bis etwa 98 Gew.-% wasserfreie Lactose,
von etwa 2 Gew.-% bis etwa 5 Gew.-% Croscarmellose-Natrium und
von etwa 1 Gew.-% bis etwa 3 Gew.-% Magnesiumstearat.

4. Tablette nach Anspruch 3, wobei das Gewichtsverhältnis der intragranulären Komponente zu der extragranulären Komponente von etwa 1:3 bis etwa 2:1 beträgt.

5. Tablette nach Anspruch 3 oder 4, wobei die Tablette durch ein Verfahren hergestellt ist, das einen Trockengranulationsprozess umfasst.

6. Tablette nach Anspruch 1, umfassend:
| | |
|---|---|
| Verbindung 1 und/oder Tautomere davon oder ein pharmazeutisch unbedenkliches Salz oder Hydrat davon | 12,5 Gew.-% |
| 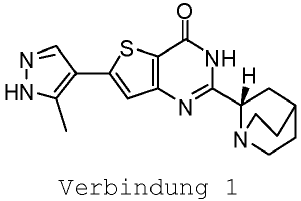 | |
| Mannit | 7,5 Gew.-% |
| silicifizierte mikrokristalline Cellulose | 3,75 Gew.-% |
| Polyvinylpyrrolidon | 0,5 Gew.-% |
| Croscarmellose-Natrium | 2,5 Gew.-% |
| Magnesiumstearat | 1,25 Gew.-% |
| wasserfreie Lactose | 72 Gew.-% |

7. Tablette nach Anspruch 1, umfassend:
| | |
|---|---|
| Verbindung 1 und/oder Tautomere davon oder ein pharmazeutisch unbedenkliches Salz oder Hydrat davon | 25 Gew.-% |
| 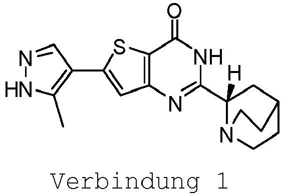 | |
| Mannit | 15 Gew.-% |
| silicifizierte mikrokristalline Cellulose | 7,5 Gew.-% |
| Polyvinylpyrrolidon | 1 Gew.-% |
| Croscarmellose-Natrium | 3 Gew.-% |
| Magnesiumstearat | 1,5 Gew.-% |
| wasserfreie Lactose | 47 Gew.-% |

## Revendications

1. Comprimé comprenant :
d'environ 10 % en poids à environ 30 % en poids d'un composé 1 et/ou des tautomères de celui-ci ou un sel ou hydrate pharmaceutiquement acceptable de celui-ci 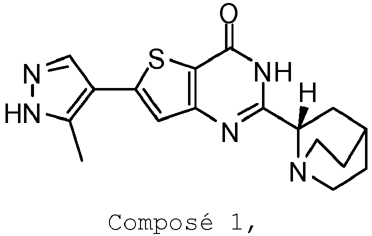
d'environ 3 % en poids à environ 10 % en poids de cellulose microcristalline silicifiée,
d'environ 5 % en poids à environ 20 % en poids de mannitol,
d'environ 0,25 % en poids à environ 2 % en poids de polyvinylpyrrolidone,
d'environ 25 % en poids à environ 80 % en poids de lactose anhydre,
d'environ 2 % en poids à environ 3 % en poids de croscarmellose sodique, et
d'environ 1 % en poids à environ 2 % en poids de stéarate de magnésium.

2. Comprimé selon la revendication 1, dans lequel le comprimé est produit par un procédé comprenans un processus de granulation sèche.

3. Comprimé comprenant un composant intra-granulaire et un composant extra-granulaire, dans lequel le composant intra-granulaire comprend :
d'environ 40 % en poids à environ 60 % en poids d'un composé 1 et/ou des tautomères de celui-ci ou un sel ou hydrate pharmaceutiquement acceptable de celui-ci 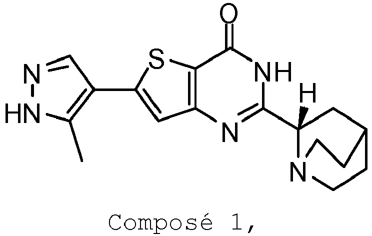
d'environ 10 % en poids à environ 20 % en poids de cellulose microcristalline silicifiée,
d'environ 20 % en poids à environ 40 % en poids de mannitol,
d'environ 1 % en poids à environ 3 % en poids de polyvinylpyrrolidone,
d'environ 1 % en poids à environ 3 % en poids de croscarmellose sodique, et
d'environ 0,5 % en poids à environ 2 % en poids de stéarate de magnésium ; et
dans lequel le composant extra-granulaire comprend :
d'environ 90 % en poids à environ 98 % en poids de lactose anhydre,
d'environ 2 % en poids à environ 5 % en poids de croscarmellose sodique, et
d'environ 1 % en poids à environ 3 % en poids de stéarate de magnésium.

4. Comprimé selon la revendication 3, dans lequel le rapport pondéral du composant intra-granulaire au composant extra-granulaire est d'environ 1:3 à environ 2:1.

5. Comprimé selon la revendication 3 ou 4, dans lequel le comprimé est produit par un procédé comprenant un processus de granulation sèche.

6. Comprimé selon la revendication 1, comprenant :
| | |
|---|---|
| Composé 1 et/ou des tautomères de celui-ci ou un sel ou hydrate pharmaceutiquement acceptable de celui-ci | 12,5 % en poids |
| 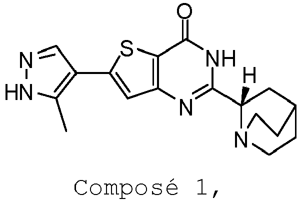 | |
| Mannitol | 7,5 % en poids |
| Cellulose microcristalline silicifiée | 3,75 % en poids |
| Polyvinylpyrrolidone | 0,5 % en poids |
| Croscarmellose sodique | 2,5 % en poids |
| Stéarate de magnésium | 1,25 % en poids |
| Lactose anhydre | 72 % en poids |

7. Comprimé selon la revendication 1, comprenant :
| | |
|---|---|
| Composé 1 et/ou des tautomères de celui-ci ou un sel ou hydrate pharmaceutiquement acceptable de celui-ci | 25 % en poids |
| 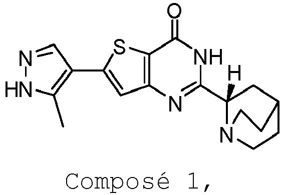 | |
| Mannitol | 15 % en poids |
| Cellulose microcristalline silicifiée | 7,5 % en poids |
| Polyvinylpyrrolidone | 1 % en poids |
| Croscarmellose sodique | 3 % en poids |
| Stéarate de magnésium | 1,5 % en poids |
| Lactose anhydre | 47 % en poids |
